Europäisches Patentamt

European Patent Office

Office européen des brevets

(11) Veröffentlichungsnummer: **0 418 635 A1**

# (12) EUROPÄISCHE PATENTANMELDUNG

(21) Anmeldenummer: 90116934.2

(22) Anmeldetag: 04.09.90

(51) Int. Cl.⁵: **G01N 33/535**, G01N 33/543, //G01N33/569,G01N33/576

(30) Priorität: 12.09.89 DE 3930376

(43) Veröffentlichungstag der Anmeldung:
27.03.91 Patentblatt 91/13

(84) Benannte Vertragsstaaten:
AT BE CH DE DK ES FR GB GR IT LI LU NL SE

(71) Anmelder: **Biotest AG**
**Flughafenstrasse 4**
**W-6000 Frankfurt am Main 73(DE)**

(72) Erfinder: **Horn, Jürgen, Dr. Dipl.-Chem.**
**Kurt-Schumacher Ring 83**
**W-6073 Egelsbach(DE)**
Erfinder: **Nebel-Schickel, Dr. Dipl.-Biol.**
**Unter den Weingärten 20**
**W-6451 Hammersbach-Marköbel(DE)**

(74) Vertreter: **Beil, Hans Christoph, Dr. et al**
**Beil, Wolff und Beil Rechtsanwälte**
**Adelonstrasse 58**
**W-6230 Frankfurt am Main 80(DE)**

(54) Enzymimmunometrisches Bestimmungsverfahren unter Verwendung von Peroxidase als Markierungsenzym.

(57) In einem enzymimmunometrischen Bestimmungsverfahren unter Verwendung von Peroxidase als Markierungsenzym lassen sich sehr geringe Mengen an zu testender Substanz, z.B. Antigen, Antikörper oder Haptene, nachweisen bzw. die Nachweisgrenze verbessern, wenn man zusammen mit dem Enzymsubstrat und dem Chromogen ein für übliche oxidierte Chromogene geeignetes Reduktionsmittel in geeigneter Konzentration verwendet.

EP 0 418 635 A1

# ENZYMIMMUNOMETRISCHES BESTIMMUNGSVERFAHREN UNTER VERWENDUNG VON PEROXIDASE ALS MARKIERUNGSENZYM.

Die Erfindung betrifft ein enzymimmunometrisches Bestimmungsverfahren nach dem Oberbegriff des Anspruchs 1.

Die Bestimmung von Peroxidase als Markierungsenzym im Rahmen der sogenannten Enzymimmuntests hat große Bedeutung erlangt. Die enzymimmunometrische Bestimmung von Haptenen, Antigenen und Antikörpern stellt ein hochempfindliches Verfahren dar. Bei der Verwendung von Peroxidase als Markierungsenzym erfolgt die Aktivitätsbestimmung durch Extinktionsmessung nach einer vorgegebenen festen Zeit.

Das Verfahren beruht auf der Oxidation eines geeigneten Chromogens in Gegenwart eines Peroxids, wobei die Geschwindigkeit der Farbbildung und die Menge des gebildeten Farbstoffs der Aktivität bzw. Menge der Peroxidase entspricht. Proben, die kein nachzuweisendes Molekül wie Antigen oder Antikörper enthalten, binden kein mit Peroxidase markiertes Antigen oder Antikörper und bleiben farblos, während Proben, die das nachzuweisende Molekül enthalten, entsprechend peroxidasemarkiertes Antigen oder Antikörper binden und daher farbig werden.

Diese Tests werden unter Verwendung einer festen Phase in der sogenannten "Sandwich"-Technik oder Modifikationen der "Sandwich"-Technik durchgeführt; d.h. generell in nichthomogenen Enzymimmuntests.

Es wird z.B. ein Antigen an einer Festphase wie Polystyrolkugeln, Polystyrollatex oder der Oberfläche von Mikrotestplatten gebunden.

Die feste Phase kann auch aus anderen, dem Fachmann bekannten, Kunststoffen oder natürlichen Polymeren bestehen; es können auch filterähnliche Festphasen entweder direkt mit Antigen beschichtet werden, oder mit Antigen an Latexpartikeln auf dem Filtermaterial verwendet werden. Die maximale Bindungskapazität ist durch die Geometrie vorgegeben.

Eventuelle freie Bindungsstellen, die nicht mit Antigen besetzt sind, können durch Serumalbumin, Makroglobuline oder sonstige nicht bei der immunologischen Reaktion beteiligten Immunglobuline abgesättigt werden oder es wird auf diese Absättigung verzichtet.

Die Konzentration des in diesem Beispiel nachzuweisenden spezifischen Antikörpers gegen das verwendete Antigen ist durch die Probe vorgegeben und kann im Frühstadium einer Infektion äußerst gering sein.

Der Nachweis erfolgt in diesem Fall bei humanem Untersuchungsgut durch einen peroxidasemarkierten Antihumanantikörper. Zur Erreichung einer höheren Nachweisempfindlichkeit kann ein möglichst affiner, möglichst hochgereinigter, hochspezifischer Antikörper ohne verunreinigendes mitmarkiertes Material eingesetzt werden. Die Empfindlichkeit läßt sich dann nach dem Massenwirkungsgesetz durch Erhöhung der Konzentration dieses markierten Antikörpers steigern. Dies ist jedoch nur in begrenztem Umfange möglich, da sonst auch bei Absättigung freier Bindungsstellen auf der Festphase mit steigender Konzentration des markierten Nachweisantikörpers ein Punkt erreicht wird, an dem auch negative Proben meßbare Extinktionen aufweisen. Diese sind dann nur noch schwierig von echt positiven, jedoch schwach positiven Proben zu unterscheiden. Insbesondere im Falle der Verwendung von Virusantigen auf der Festphase, nachzuweisendem Serumantikörper und markiertem anti-Spezies-Antikörper ist die unspezifische Bindung in der Regel so groß, daß die nachzuweisenden Serumantikörper in aller Regel in Puffer verdünnt werden müssen, wobei die bekannten Testsysteme Vorverdünnungen von 1:5 bis über 1:100 verwenden.

Der Erfindung liegt die Aufgabe zugrunde, ein enzymimmunometrisches Verfahren unter Verwendung von Peroxidase als Markierungsenzym bereitzustellen, das es erlaubt, sehr geringe Mengen an zu testender Substanz, z.B. Antigen, Antikörper oder Haptene, nachzuweisen bzw. die Nachweisempfindlichkeit zu verbessern.

Diese Aufgabe wird erfindungsgemäß bei einem gattungsgemäßen Verfahren durch die kennzeichnenden Merkmale des Anspruchs 1 gelöst.

Die Reduktion von peroxidaseoxidierten Phenolen zu ihren nichtoxidierten Ausgangsverbindungen mit Hilfe von Ascorbaten in einem homogenen System ist aus J.Biochem. 102, 785-791 (1987) bekannt. Hier wird der Unterschied der Peroxidaseaktivität zwischen freier und aggregierter (polymerisierter) Peroxidase, die durch überschüssiges $H_2O_2$ inhibiert wird, untersucht. Da dabei die Farbbildung infolge Oxidation so stark ist, daß man das eigentliche Spektrum der sich bildenden Zwischenprodukte nicht mehr erkennen kann, wird die Peroxidaseaktivität als Farbbildungsverzögerungszeit in Gegenwart von Ascorbat gemessen.

Bei der erfindungsgemäßen Verwendung eines Reduktionsmittels für Chromogene in einem nicht homogenen System war es jedoch völlig überraschend, daß sich die Testempfindlichkeit verbessern und das Signal von empfindlichen Proben anheben ließ, ohne dabei das Signal von negativen Proben anzuheben.

Die vorliegende Erfindung erlaubt es, wesentlich höhere Konzentrationen an markiertem anti-Spezies-Antikörper einzusetzen, wobei negative Proben in der Extinktion negativ bleiben, positive jedoch eine wesentlich höhere Extinktion ergeben. Dies erlaubt, positive Proben weiter mit negativem Serum oder Plasma zu verdünnen, um eine echte Probe sehr niedrigen Gehaltes an spezifischem z.B. Virusantikörper zu imitieren.

Diese Probe ist im normalen Testverfahren negativ, mit der vorliegenden Erfindung jedoch deutlich positiv. Weiter ist der unspezifische Hintergrund so absenkbar, daß mit gleicher oder auch wesentlich verbesserter Empfindlichkeit Serum oder Plasma einsetzbar ist, welches nicht mit Puffer verdünnt wurde.

Die zu verwendenden Virusantigene sind ihrer Art nach nicht beschränkt, es können alle Virusantigene, ob natürlich gewonnen oder als rekombinante Proteine oder Proteinbruchstücke oder Peptide, verwendet werden. Die gleichen Verbesserungen sind mit dem erfindungsgemäßen Verfahren auch mit anderen, dem Fachmann bekannten, nichthomogenen Enzymimmuntestverfahren möglich.

Es kann eine Festphase mit spezifischen Antikörpern gegen ein Virusantigen oder bakterielles Antigen oder ein beliebiges als Antigen wirkendes Protein beschichtet werden, wobei alle dem Fachmann bekannten Variationen der Festphase möglich sind. Die Untersuchungsprobe, z.B. Serum oder Plasma, enthält das nachzuweisende Antigen, z.B. Virusantigen. Zum Nachweis wird ein peroxidasemarkierter spezifischer Antikörper gegen das nachzuweisende Antigen eingesetzt, wobei erfindungsgemäß wesentlich höhere Konzentrationen und verbesserte Nachweisgrenzen (Sensitivität) möglich sind.

Die Festphase kann mit anti-Immunglobulinklassenantikörpern wie anti-IgM beschichtet sein. Die Untersuchungsprobe ist Serum oder Plasma, die den nachzuweisenden Antikörper enthält. Zum Nachweis dient ein peroxidasemarkiertes Virusantigen, wobei erfindungsgemäß wesentlich höhere Konzentrationen und verbesserte Nachweisgrenzen möglich sind. Dies gilt auch für Modifikationen, wie Verwendung eines biotinmarkierten Antigens oder biotinmarkierten Nachweisantikörpers, wobei der eigentliche farbgebende Nachweis in diesem Falle mit peroxidasemarkiertem Streptavidin bzw. Avidin erfolgt. Ebenfalls für die umgekehrte Modifikation mit biotinmarkierter Peroxidase und dann entsprechend streptavidin-/avidinmarkiertem Antigen bzw. streptavidinmarkiertem Antikörper.

Die erfindungsgemäße Bestimmung von Peroxidase mit reduziertem background und verbesserter Empfindlichkeit erfolgt durch Zugabe geringer Mengen eines geeigneten Reduktionsmittels zu der verwendeten Substratlösung aus Peroxidase und Chromogen, wie ortho-Phenylendiamin, Tetramethylbenzidin oder ähnlichen aromatischen Amin/Phenol-Chromogenen.

Geeignete Reduktionsmittel sind alle wasserlöslichen Reduktionsmittel, die in der Lage sind, die als Chromogene eingesetzten oxidierten aromatischen Amine und Phenole zu reduzieren. Beispiele dafür sind Ascorbinsäure, wasserlösliche Ascorbate, Thiosulfate, Hydrogensulfite, Natriumdithionit, Cysteinhydrochlorid, Mercaptoethylaminhydrochlorid, Dithiothreitol, weitere Mercapto- und Dithioverbindungen, Mischungen von Dithio- und Mercaptoverbindungen, Mischungen von Cephalosporin C und Mercaptoverbindungen, oder Cephalosporin C und Ascorbat sowie Mischungen von Ascorbat (Ascorbinsäure + Salz) und Mercaptoverbindungen. Besonders bevorzugt sind Ascorbinsäure und Ascorbate.

Das Reduktionsmittel reduziert gebildeten Farbstoff zur farblosen Ausgangsverbindung und führt somit zu einem verzögerten Einsetzen der Reaktion. Die Konzentration des Reduktionsmittels muß geeignet niedrig gewählt werden, um während des Ablaufs der Enzymreaktion über eine vorbestimmte Zeit den (durch erhöhte Konzentration an peroxidasemarkiertem Nachweisantikörper/-antigen) unspezifischen background bei negativen Proben niedrig zu halten und jedoch gleichzeitig zu ermöglichen, positive Proben sehr niedriger Konzentration (die bei normaler Konzentration an Nachweisantikörper/-antigen negativ erscheinen würden) noch eindeutig positiv werden zu lassen.

Da das gegenüber dem Reduktionsmittel wesentlich teurere Reagenz der peroxidasemarkierte Nachweisantikörper/-antigen ist, wird die Sensitivitätssteigerung mit möglichst geringer Erhöhung an markiertem Nachweisantikörper/-antigen und entsprechend geringer Konzentration an Reduktionsmittel durchgeführt. Es können natürlich auch noch wesentlich höhere Konzentrationen an Nachweisantikörper/-antigen eingesetzt werden, mit entsprechender Konzentration an Reduktionsmittel. Die Konzentration des Reduktionsmittels wird weiter beeinflußt, wenn Maßnahmen wie Absättigung freier Oberflächen der Festphase durch nicht an der immunologischen Reaktion beteiligte Makromoleküle, wie Serumalbumin oder ähnliches, getroffen wurden. Die Konzentrationen sind ferner durch Absorptionsverhalten der Festphase und Menge an verwendeter gebundener Peroxidase zu beeinflußen. Um die optimale Menge an Reduktionsmittel zu ermitteln, ist eine Versuchsreihe mit verschiedenen Konzentrationen an Reduktionsmittel durchzuführen. Dies ist eine jedem Fachmann geläufige und zuzumutende Maßnahme, die keiner erfinderischen Leistung bedarf.

Die optimale Konzentration liegt vorzugsweise in einem Bereich von 0,01 bis 0,5 mM.

Das Substrat für Peroxidase ist $H_2O_2$. Somit wird im vorliegenden unter Enzymsubstrat $H_2O_2$ verstanden. Gewöhnlich liegt das Substrat in gepufferter Lösung vor. Der Begriff Substrat-Puffer umfaßt somit $H_2O_2$ in gepufferter Lösung. Besonders geeignet ist ein Citrat-Phosphat-Puffer.

Das Reduktionsmittel kann der Substrat bzw. Substrat-Puffer/Chromogenlösung zur Aktivitätsbestimmung direkt vor Reaktion zugesetzt werden oder bereits im Substrat-Puffer enthalten sein als Bestandteil des Substrat-Puffers oder bereits im Chromogen (liegt in der Regel als Pulver oder Reagenztablette vor) enthalten sein.

Nachstehende Beispiele dienen der weiteren Erläuterung der Erfindung.

Bestimmung von anti-CMV-IgG mit Testkit Biotest CMW IgG mit Standardkomponenten und parallel mit erhöhter Konzentration an peroxidasemarkiertem Nachweisantikörper ohne und mit Reduktionsmittel. Der markierte Antikörper ist BS12, Maus anti human IgG.

Die Bestimmungsmethode wird in Biotest Anti-CMV IgG ELISA, Enzymimmunoassay zum Nachweis von IgG-Antikörpern gegen das Zytomegalievirus, zugelassen vom Paul-Ehrlich-Institut (Zul.-Nr. 62a/86), näher beschrieben, dessen Kopie als Anlage beigefügt wird und Bestandteil der vorliegenden Offenbarung sein soll.

Beispiel 1

| | 1 | 2 | 3 |
|---|---|---|---|
| peroxidasemarkierter Nachweisantikörper | Charge 88-296-OF BS-12-Peroxidase 0,2 µg/ml | Charge 88-296-OF BS-12-Peroxidase 1,0 µg/ml | Charge 88-296-OF BS-12-Peroxidase 1,0 µg/ml |
| Substrat-Puffer | Standard, ohne Reduktionsmittel | Standard, ohne Reduktionsmittel | erfindungsgemäß mit 0,15 milli M Ascorbinsäure |
| Probe | OD = optische Dichte (Differenz zwischen CMV-Antigen und Kontrollantigen) | | |

| Konzentration an anti CMV IgG (nach CMV-Standard PEI) | | | |
|---|---|---|---|
| 0,05 U/ml | 0,160 | 1,296 | 0,856 |
| 0,1 U/ml | 0,502 | 2,154 | 1,949 |
| 0,2 U/ml | 0,924 | 2,591 | 2,969 |
| 0,4 U/ml | 1,212 | 2,852 | 2,989 |
| negative Kontrolle (0 U/ml) 85-081-OF | 0,002 | 0,104 | 0,000 |

Die Proben wurden unverdünnt eingesetzt (Einstellung der positiven Serumproben durch Verdünnung mit negativem Serum, es erfolgt keine weitere Verdünnung der Proben mit Puffer

Zu 1) Standard-Testkit mit niedriger Konzentration des enzymmarkierten Konjugats, relativ niedrige Signale;

Zu 2) Standard-Testkit mit erhöhter Konzentration, Signale zwar höher, jedoch auch bei negativen Kontrollproben;

Zu 3) Erfindungsgemäß mit erhöhter Konzentration und Einsatz eines Reduktionsmittels, erhöhte Signale der positiven Proben, wobei negative Kontrollproben negativ bleiben.

Beispiel 2

| peroxidasemarkierter Nachweisantikörper | *1* Charge 87-263-OF BS-12-Peroxidase 0,3 µg/ml | *2* Charge 87-263-OF BS-12-Peroxidase 5 µg/ml | *3* Charge 87-263-OF BS-12-Peroxidase 5 µg/ml |
|---|---|---|---|
| Substrat-Puffer | Standard, ohne Reduktionsmittel | Standard, ohne Reduktionsmittel | erfindungsgemäß mit 0,1 milli M Ascorbinsäure |
| Probe | OD = optische Dichte (Differenz zwischen CMV Antigen und Kontrollantigen). | | |

Konzentration von
anti CMV IgG
(nach CMV Standard
Paul-Ehrlich-Institut)

| | 1 | 2 | 3 |
|---|---|---|---|
| negative Kontrolle (0 U/ml) | 0,017 | 0,141 | 0,018 |
| 0,2 U/ml | 0,315 | 2,695 | 2,588 |
| 0,1 U/ml | 0,123 | 2,212 | 2,105 |
| 0,05 U/ml | 0,051 | 1,426 | 1,310 |
| 0,025 U/ml | 0,028 | 1,108 | 0,967 |
| 0,0125 U/ml | 0,020 | 0,788 | 0,612 |
| 0,00625 U/ml | 0,021 | 0,435 | 0,365 |

Die proben wurden alle 1:5 verdünnt mit Puffer eingesetzt (positive Serumproben wurden durch Verdünnen mit negativem Serum eingestellt, bevor die Testverdünnung durch Puffer durchgeführt wurde).

Als positiv gelten alle Proben mit einer OD ≥0,200. D.h. die Nachweisgrenze des unmodifizierten Tests (Standardprodukt) liegt bei 0,2 U/ml, die Modifikation mit erhöhter Konzentration an peroxidasemarkiertem Nachweisantikörper und Reduktionsmittel bei der Enzymreaktion liegt bei 0,00625 U/ml, d.h. eine Steigerung um den Faktor 30. Bei erfindungsgemäßem Vorgehen steigt die optische Dichte der negativen Kontrolle nicht an (bei dem wesentlich erhöhten Wert der negativen Kontrolle bei erhöhter Konzentration an BS12-Peroxidase und ohne Reduktionsmittel müßte ein viel höherer Wert als 0,200 als Grenzwert zur Unterscheidung positiv/negativ festgelegt werden).

Die Modifizierung des Standardtests mit dem Einsatz von unverdünnten Proben (mit background-Unterdrükkung durch Nachbeschichten der Festphase) ergibt eine Nachweisgrenze von 0,1 U/ml. Die erfindungsgemäße Modifizierung mit leicht erhöhter Konzentration an peroxidasemarkiertem Nachweisantikörper und Reduktionsmittel, läßt eine Nachweisgrenze von 0,025 bis 0,0125 U/ml extrapolieren, d.h. eine Steigerung der Empfindlichkeit um den Faktor 4 bis 8 bei nur mäßig erhöhten Kosten durch den geringen Konzentrationsanstieg. Weiter kann hier bei dem Prinzip des Nachweises von Serumantikörpern an Virusantigen an Festphase mit markiertem anti-Spezies-Antikörper auf die Vorverdünnung mit Puffer verzichtet werden.

Empfindlichkeitssteigerung beim Nachweis von anti HIV Verwendet werden Mikrotestplatten beschichtet mit rekombinantem p 24 und gp 41 Proteinen.

Beispiel 3

| Testantigen | p 24 | p 24 | p 24 | gp 41 | gp 41 | gp 41 |
|---|---|---|---|---|---|---|
| Plattencharge | 8/22 | 8/22 | 8/22 | 8/14 | 8/14 | 8/14 |
| Nachweisanti-körpercharge | BS-12 POD 88-296-OF | BS-12 POD 88-296-OF | BS-12 POD 88-296-OF | BS-12 POD 88-296-OF | BS-12 POD 88-296-OF | BS-12 POD 88-296-OF |
| Konzentration | 0,2 µg/ml | 1,0 µg/ml | 1,0 µg/ml | 0,2 µg/ml | 1,0 µg/ml | 1,0 µg/ml |
| Substrat-Puffer | Standard | Standard | mit 0,15 milli M Ascorbins. | Standard | Standard | mit 0,15 milli M Ascorbins. |

Probe unterschiedlicher Spender      * OD = optische Dichte

| | p 24 | p 24 | p 24 | gp 41 | gp 41 | gp 41 |
|---|---|---|---|---|---|---|
| 1.) | 0,007 | 0,076 | 0,004 | 0,013 | 0,122 | 0,005 |
| *2.) | 0,345 | 1,435 | 0,797 | 1,646 | >3,0 | 2,773 |
| | 0,140 | 0,689 | 0,155 | 0,821 | >3,0 | 2,278 |
| | 0,048 | 0,295 | 0,014 | 0,309 | 1,372 | 0,788 |
| | 0,017 | 0,157 | 0,005 | 0,109 | 0,625 | 0,169 |
| 3.) | 0,034 | 0,240 | 0,013 | >3,0 | >3,0 | >3,0 |
| 4.) | 0,024 | 0,175 | 0,009 | 0,120 | 0,581 | 0,151 |
| 5.) | 0,015 | 0,159 | 0,034 | 0,026 | 0,216 | 0,015 |
| 6.) | 0,004 | 0,074 | 0,030 | 0,032 | 0,252 | 0,022 |
| 7.) | 0,006 | 0,075 | 0,021 | 0,121 | 0,646 | 0,144 |
| 8.) | 0,000 | 0,033 | 0,000 | 1,206 | >3,0 | >3,0 |
| 9.) | 0,011 | 0,110 | 0,006 | 0,005 | 0,102 | 0,004 |
| 10.) | 0,006 | 0,073 | 0,005 | 0,018 | 0,080 | 0,008 |
| 11.) | 0,327 | 1,522 | 0,898 | 0,012 | 0,135 | 0,004 |

*es handelt sich um Titrationsreihen der positiven Probe 2

Alle Proben 1:21 in Puffer verdünnt.
Empfindlichkeitssteigerung beim Nachweis von IgG-Antikörpern und IgM-Antikörpern gegen EBV

Beispiel 4

| Testantigen | EBV p 150 (VCA) | p 150 | p 150 |
|---|---|---|---|
| Nachweisanti- körpercharge | BS-12-POD 88-296-OF | BS-12-POD 88-296-OF | BS-12-POD 88-296-OF |
| Konzentration | 0,2 µg/ml | 1,0 µg/ml | 1,0 µ/ml |
| Substrat-Puffer | Standard | Standard | mit 0,05 milli M Ascorbinsäure |

* OD = optische Dichte

| Probe unterschied- licher Spender | | | |
|---|---|---|---|
| 12.) | 0,191 | 1,737 | 1,471 |
| 13.) | 0,058 | 0,517 | 0,492 |
| 14.) | 0,228 | 0,761 | 1,900 |
| 15.) | 0,012 | 0,172 | 0,041 |
| 16.) | 1,792 | 2,674 | >3,0 |
| 17.) | 0,165 | 1,373 | 1,304 |
| 18.) | 0,125 | 1,062 | 1,270 |
| 19.) | 0,024 | 0,274 | 0,063 |

Alle Proben 1:21 in Puffer verdünnt.
Negative Proben wie 15.) und 19.) bleiben eindeutig negativ, das positive Signal von positiven Proben wie 12.), 17.), 18.) 16.) wird wesentlich verstärkt.

EP 0 418 635 A1

Beispiel 5

| Testantigen | EBV p 150 (VCA) | p 150 | p 150 |
|---|---|---|---|
| Nachweisanti-körpercharge | anti-IgM-POD MH 15-01-ME4. | anti-IgM-POD MH 15-01-ME4 | anti-IgM-POD MH 15-01-ME4 |
| Konzentration | 0,75 µg/ml | 0,5 µg/ml | 0,5 µg/ml |
| Substrat-Puffer | Standard | Standard | mit 0,15 milli M Ascorbinsäure |

Probe unterschiedlicher Spender — *OD = optische Dichte

| | | | |
|---|---|---|---|
| 20.) | 0,021 | 0,218 | 0,003 |
| 21.) | 0,337 | 2,036 | 1,796 |
| 22.) | 0,037 | 0,226 | 0,036 |
| 23.) | 0,075 | 0,513 | 0,227 |
| 25.) | 0,071 | 0,585 | 0,330 |
| 25.) | 0,015 | 0,154 | 0,001 |

Alle Proben 1:21 in Puffer verdünnt und alle Proben mit Rheumafaktorabsorbens vorabsorbiert.

Negative Proben wie 20.), 25.), 22.) sind mit erhöhter Konjugatkonzentration ohne erfindungsgemäße Substratzusammensetzung falsch erhöht, mit Reduktionsmittel dagegen weiter richtig negativ.

Positive Proben wie 21.) werden im Signal stark angehoben. Schwach positive Proben wie 23.), 24.) werden nur durch die erhöhte Konjugatkonzentration positiv und sind durch erfindungsgemäße Substratzusammensetzungen einwandfrei von negativen Proben zu differenzieren.

Empfindlichkeitssteigerung beim Nachweis von HBs Ag

8

Beispiel 6

| Testantikörper | anti HBs Meerschwein | anti HBs Meerschwein | anti HBs Meerschwein |
|---|---|---|---|
| Nachweisanti-körper | anti HBs-POD Ziege | anti HBs-POD Ziege | anti HBs-POD Ziege |
| Konzentration | 0,15 µg/ml | 2,5 µg/ml | 2,5 µg/ml |
| Substrat-Puffer | Standard | Standard | mit 0,1 milli M Ascorbinsäure |

\* OD = optische Dichte

| Probe | | | |
|---|---|---|---|
| HBs Ag ad 1 ng/ml | 1,022 | >3,0 | >3,0 |
| 0,3 ng/ml | 0,306 | 2,285 | 2,131 |
| 0,1 ng/ml | 0,057 | 1,672 | 1,458 |
| 0,03 ng/ml | 0,016 | 0,985 | 0,812 |
| 0,01 ng/ml | 0,002 | 0,412 | 0,299 |
| HBs Ag ay 1 ng/ml | 1,075 | >3,0 | >3,0 |
| 0,3 ng/ml | 0,289 | 2,391 | 2,182 |
| 0,1 ng/ml | 0,055 | 1,738 | 1,516 |
| 0,03 ng/ml | 0,018 | 1,031 | 0,852 |
| 0,01 ng/ml | 0,001 | 0,488 | 0,317 |
| negative Kontrolle | 0,002 | 0,298 | 0,009 |

Durch das erfindungsgemäße Vorgehen mit erhöhter Konzentration an markiertem Nachweisantikörper und Reduktionsmittel im Substrat wird eine deutliche Signalanhebung und Verbesserung der Testempfindlichkeit von 0,1 ng/ml auf unter 0,01 ng/ml erreicht.

In den Beispielen 1 bis 6 wurden als chromogenes Substrat jeweils 30 mg OPD-Orthophenylendiamin in 10 ml Substrat-Puffer verwendet.

## Beispiel 7

```
Testantigen        :  gp41
Nachweisantikörper:  BS12-POD
                     Charge 88-356-OF
```

| Konzentration des Nachweisantikörpers | 0,08 µg/ml | 0,3 µg/ml | 0,3 µg/ml | 0,3 µg/ml |
|---|---|---|---|---|
| Substrat-Puffer | Standard | Standard | mit 0,05mM Cystein | mit 0,05mM Cystein + 0,01mM Dithio-threitol |

| Probe unterschied-licher Spender (OD = optische Dichte) | | | | |
|---|---|---|---|---|
| 1.) | 0,012 | 0,088 | 0,006 | 0,004 |
| ✳ 2.) | 0,830 | 1,650 | 1,620 | 1,589 |
| | 0,485 | 0,962 | 0,990 | 1,028 |
| | 0,138 | 0,468 | 0,532 | 0,502 |
| 3.) | >2,0 | >2,0 | >2,0 | >2,0 |
| 4.) | 0,162 | 0,590 | 0,562 | 0,570 |
| 8.) | 1,105 | >2,0 | >2,0 | >2,0 |
| 10.) | 0,018 | 0,129 | 0,007 | 0,006 |

* es handelt sich um Titrationsreihen der positiven Probe 2

In Beispiel 7 wurde als chromogenes Substrat 2 mg TMB = Tetramethylbenzidin in 10 ml Substrat-Puffer verwendet.

Der Substrat-Puffer-Standard bestand in allen Beispielen aus 0,1 molarem Citrat/Phosphatpuffer mit 0,02 % $H_2O_2$, erfindungsgemäß enthielt er jeweils noch zusätzlich die in den Tabellen angegebenen Zusätze.

Die in den Tabellen verwendeten Abkürzungen haben folgende Bedeutung:

BS12-POD steht für peroxidasemarkiert (POD) anti-human-IgG-Antikörper, monoklonale, Hersteller Biotest AG (Seriennummer BS12)

HBs oder HBsAg = Hepatitis B surface Antigen

HIV = Human immunodeficiency virus identisch mit HTLV III (alte Bezeichnung human T lymphotropic virus III)

Erläuterungen zu den HIV-Antigenen p24 und gp41 finden sich in den Literaturstellen:

British Medical Journal, Vol. 294, 20th June 1987, p.1602-1605,

Biotechnology, Vol. 6, Number 3, March 1988, p. 259-264, Viruses and Human Cancer, 1987, Alan R. Liss, Inc., pages 15-28.

EBV-Antigen

p54 entspricht dem Leserahmen BMRF1 des B95-8 Epstein Barr Virus Genoms

p138 entspricht dem Leserahmen BALF2 des B95-8 Epstein Barr Virus Genoms

p150 entspricht dem Leserahmen BcLF1 des B95-8 Epstein Barr Virus Genoms

p72 entspricht dem Leserahmen BKRF1 des B95-8 Epstein Barr Virus Genoms

Die zu diesen (reading frames) Leserahmen zugehörigen Literaturstellen sind folgende:

Baer, R., et al.: DnA sequence and expression of the B95-8 Epstein-Barr virus genome. Nature 310 , 207-211 (1984).

Dillner, J., and Kallin, B.: The Epstein-Barr virus proteins". in: "Advances in Cancer Research", Vol. 50 , 95-158, Klein, G., and Weinhouse, S. (eds.), Academic Press, San Diego (1988).

Thoxley-Lawson, D.A.: Immunological responses to Epstein-Barr virus infection and the pathogenesis of EBV-induced diseases". Biochim. Biophys. Acta 948 , 263-286 (1988).

EP 0 418 635 A1

Pearson, G.R.: "ELISA tests and monoclonal antibodies for EBV". J. Virol. Meth. 21 , 97-104 (1988).
Zu p138: Motz, M., Fan, J., Seibl, R., Jilg, W., and Wolf, H.: Expression of the Epstein-Barr virus 138 kDa
early protein in Escherichia coli for the use as antigen in diagnostic test. Gene 42 , 303-312 (1986).
Hinderer, W., Nebel-Schickel, H., Sonneborn, H.-H., Motz, M., Kühbeck, R., Wolf, H.: Purification of four
different recombinant EBV-antigens synthesized in E.coli and their diagnostic application. J. Exp., Cancer
Res. 7, 132 (1988).
Middledorp, J.M., and Melsen, R.H.: Epitope-mapping on the Epstein-Barr virus major capsid protein using
systematic synthesis of overlapping oligopeptides. J. Virol. Meth. 21 , 147-159 (1988).

Beschreibung der Herstellung der verwendeten EBV-Platten bzw. HIV-Platten bzw. anti-HBs-Platten:

EBV-Platten:

EBV-Antigen p150 wird in einer Konzentration von 2 Mikrogramm/Milliliter in 0,01 molarem Carbonatpuffer pH 9,5 je 100 mikroliterweise in Vertiefungen einer Polystyrol-Mikrotestplatte (Firma Nunc) gefüllt und über Nacht bei Raumtemperatur stehen gelassen. Nach Ausleeren wird 2 mal mit 0,01 molarer phosphatgepufferter Kochsalzlösung pH 7,2 gewaschen.

HIV-Platten:

HIV-Antigene gp41 bzw. p24 werden in einer Konzentration von 2 Mikrogramm/Milliliter in 0,01 molarem Carbonatpuffer pH 9,5 je 100 mikroliterweise in Vertiefungen einer Polystyrol-Mikrotestplatte (Firma Nunc) gefüllt und über Nacht stehen gelassen. Nach Ausleeren wird 2 mal mit 0,01 molarer phosphatgepufferter Kochsalzlösung pH 7,2 gewaschen.

anti-HBs-Platten:

Meerschweinchen werden mit gereinigtem HBsAg immunisiert, und die IgG-Fraktion aus dem Serum der immunisierten Meerschweinchen wird durch Fällung mit Ammoniumsulfat gewonnen (Practical Immunology, Blackwell Scientific Publications, L. Hudson, ISBN 0632002115); anti-HBs wird in einer Konzentration von 1 Mikrogramm/Milliliter in 0,01 molarem Carbonatpuffer pH 9,5 je 100 mikroliterweise in Vertie fungen einer Polystyrol-Mikrotestplatte (Firma Nunc) gefüllt und über Nacht stehen gelassen. Nach Ausleeren wird 2 mal mit 0,01 molarer phosphatgepufferter Kochsalzlösung pH 7,2 gewaschen.

**Ansprüche**

1. Enzymimmunometrisches Bestimmungsverfahren, bei dem man einen zu bestimmenden Antikörper, ein Antigen oder Hapten immunologisch an eine Festphase bindet und zum Nachweis einen peroxidasemarkierten Nachweisantikörper, -antigen oder -hapten verwendet, wobei man die immunologisch gebundene Peroxidase durch Zugabe von Enzymsubstrat und Chromogen nachweist, **dadurch gekennzeichnet,** daß man zusammen mit dem Enzymsubstrat und dem Chromogen ein für übliche oxidierte Chromogene geeignetes Reduktionsmittel in geeigneter Konzentration verwendet.
2. Verfahren nach Anspruch 1, dadurch gekennzeichnet, daß man als Reduktionsmittel Ascorbinsäure oder ein wasserlösliches Ascorbat verwendet.
3. Verfahren nach Anspruch 1 oder 2, dadurch gekennzeichnet, daß die Konzentration an verwendetem Reduktionsmittel 0,01 bis 0,5 mM beträgt.
4. Verfahren nach einem der vorstehenden Ansprüche, dadurch gekennzeichnet, daß die Peroxidase an Antikörper, Antigene, Haptene, Biovitin oder Avidin gebunden ist.
5. Verfahren nach einem der vorstehenden Ansprüche, dadurch gekennzeichnet, daß das Reduktionsmittel im Substrat oder im Chromogen vorliegt oder daß es der Substrat/Chromogenlösung direkt vor der Reaktion zugesetzt wird.
6. Kit zur Durchführung des Verfahrens nach einem der Ansprüche 1 bis 5, enthaltend eine Festphase, einen peroxidasemarkierten Nachweisantikörper, -antigen oder -hapten, ein Substrat, vorzugsweise in einer Pufferlösung, ein Chromogen und ein Reduktionsmittel für das oxidierte Chromogen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 202 081  (SYNTES (USA) INC.) <br> * Seite 2, Zeile 18 - Seite 3, Zeile 23; Seite 7, Zeilen 24-36; Seite 15, Zeilen 9-18; Seite 19, Zeilen 6-9; Seite 21, Zeilen 13-24; Seite 26, Zeile 33 - Seite 27, Zeile 36 * <br> – – – | 1-6 | G 01 N 33/535 <br> G 01 N 33/543 // <br> G 01 N 33/569 <br> G 01 N 33/576 |
| X | REVISTA ESPANOLA DE FISIOLOGIA, Band 45, Nr. 1, Januar-März 1989, Seiten 41-46; J.M. COLL et al.: "Addition of reducing agents to the peroxidase-o-phenylenediamine buffer reduces background of enzyme immunoassays" <br> * Seiten 41-43 * <br> – – – | 1-6 | |
| A | WO-A-8 808 536  (BOOTS-CELLTECH DIAGNOSTICS LTD) <br> * Seite 3, Zeile 24 - Seite 5, Zeile 22; Seite 8, Zeilen 1-32 * <br> – – – | 1,2,4-6 | |
| A | J. CLIN. CHEM. CLIN. BIOCHEM., Band 23, 1985, Seiten 41-44; T. PORSMANN et al.: "Stabilization of the substrate reaction of horseradish peroxidase with o-phenylenediamine in the enzyme immunoassay" <br> * Zusammenfassung * <br> – – – – – | 1,4 | |

| | | | RECHERCHIERTE SACHGEBIETE (Int. Cl.5) |
|---|---|---|---|
| | | | G 01 N |

**Der vorliegende Recherchenbericht wurde für alle Patentansprüche erstellt**

| Recherchenort | Abschlußdatum der Recherche | Prüfer |
|---|---|---|
| Den Haag | 19 Dezember 90 | HITCHEN C.E. |